# EUROPEAN PATENT APPLICATION

(11) **EP 3 991 732 A1**
(43) Date of publication of application: **04.05.2022**
(21) Application number: 20204769.2
(22) Date of filing: 29.10.2020
(51) Int. Cl.: A61K 31/498, A61K 45/06, A61P 35/00, G01N 33/50

(54) **USE OF CLOFAZIMINE IN HEPATOCELLULAR CARCINOMA**

(71) Applicant: UNIVERSITE DE GENEVE, 1211 Geneva 4 (CH)
(72) Inventor: KATANAEV, Vladimir, 1260 NYON (CH)
(74) Representative: reuteler & cie SA

(57) **Abstract**

The present invention relates to clofazamine as well as pharmaceutically acceptable salts thereof, for use in the treatment of HCC in a subject, wherein said subject presents: an overactive wnt signalling; and a genetic background of Hepatitis B virus (HBV) infection. This invention provides related methods, formulations and uses thereof.

## Description

### Field of the Invention

The present invention relates to the use of clofazimine in Hepatocellular Carcinoma (HCC).

### Background of the Invention

Wnt signaling plays key roles in oncogenic transformation and progression in a number of cancer types, including tumors in the breast, colon, ovaries, liver, and other tissues (Ghosh et al., 2019,Ann N Y Acad Sci, 1443, 54-74). Despite this importance, no therapy targeting the Wnt pathway currently exists and many of the anti-Wnt compounds failed to show an acceptable safety profile, mainly due to the function of the adult Wnt pathway in controlling tissue renewal and regeneration in the adult organs (Shaw et al., 2019b, Swiss Med Wkly 149, w20129.*;* Kahn, 2014, Nature Reviews Drug Discovery, 13, 513-532).

Clofazimine (IUPAC name: *N*,5-bis(4-chlorophenyl)-3-(propan-2-ylimino)-3,5-dihydrophenazin-2-amine) is a riminophenazine derivative a fully synthetic product based on a chemical scaffold initially obtained from lichens (Reddy et al., 1999, Journal of Antimicrobial Chemotherapy, 43(5), 615-623) was first synthesized and tested against tuberculosis *(*Barry et al., 1957, Nature 179, 1013-1015*),* however, its efficacy against *M. tuberculosis* was considered insufficient. Instead clofazimine demonstrated to be more potent against another mycobacterium, *M. leprae* (Browne and Hogerzeil, 1962, Lepr Rev 33, 6-10). Thus, since early 1960s clofazimine was remarketed as for the treatment of leprosy and is recommended by WHO as part of the standard multidrug therapy of leprosy (https://apps.who.int/iris/handle/10665/64636). Clofazimine's efficacy in treating leprosy because was attrributed not only to its antimicrobial activity but also anti-inflammatory activity *(*Singh et al., 2016, J Infect Dis, 214, 1456-1464*).* Clofazimine is further reported to be useful for several infectious and non-infectious diseases, such as atypical mycobacterial infections, rhinoscleroma, pyoderma gangrenosum, necrobiosis lipoidica, severe acne, pustular psoriasis, and discoid lupus erythematosus (Arbiser and Moschella, 1995, JAm Acad Dermatol 32, 241-247). Beginning from the mid-90s arise studies testing the anticancer potentials of clofazimine, showing that it is effective against cultures of squamous hepatocellular carcinoma *(Van* Rensburg et al., 1993, Cancer Res 53, 318-323*)* and lung cancer *(*Sri-Pathmanathan et al., 1994, Int J Cancer, 56, 900-905*)* cell lines. However, few mechanistic studies on this effect have been done (*Ahmed et al., 2019, supra*). Moreover, clofazimine was tested for its anticancer activity against advanced hepatocellular carcinoma, however, without a clear positive outcome in this indication (Ruff et al., 1998, Ann Oncol 9, 217-219*;*Falkson and Falkson, 1999, Oncology 57, 232-235).

Hepatocellular carcinoma (HCC) is the sixth most diagnosed cancer worldwide and the third leading cause of cancer-associated death *(*Forner et al., 2018, Lancet, 391, 1301-1314*).*

Currently, there are an estimated 29,200 new HCC cases in males and 11,510 cases in females in the United States in 2017, among which 29,000 cases died from HCC (Siegel et al., 2017, CA: A Cancer Journal for Clinicians, 67(1), 7-30*).* More seriously, estimated new HCC cases achieved 343,700 in males and 122,300 in females in China in 2015 (Chen et al., 2016, A Cancer Journal for Clinicians, 66(2), 115-132*),* which is mainly attributed to the prevalence of hepatitis B virus (HBV) persistent infection and HBV-induced cirrhosis (*Ren et al., 2020, ID 8157406* | *https:*//*doi.org*/*10.1155*/*2020*/*8157406*)*.*

In Africa and Asia, where Hepatitis B virus (HBV) is endemic, ca. 60% of HCC is associated with HBV, ca. 20% is related to HCV and the remainder is distributed among other risk factors (for example, alcohol and aflatoxin) (Arzumanyan et al., 2013, NATURE REVIEWS : Cancer, 13, 123-135*).* In Europe and the USA, only ca. 20% of HCC is associated with HBV.

Due to the absence of specific symptoms in early stages, the lack of early diagnostic markers, and the low percentage (10-20%) of radical resectable HCC on diagnosis, most HCC patients are often diagnosed in an advanced stage with poor prognosis (the overall ratio of mortality to incidence is 0.95) (Kobayashi et al., 2017, Hepatobiliary & Pancreatic Diseases International, 16(3), 279-288)*.* Most treatments are inadequate and mortality is high. Patients with small, single tumours are treated by surgical resection or liver transplantation, but relapse is common *(Arzumanyan et al., 2013, supra).* Further, although liver transplantation (LT) has been considered the most effective therapy for HCC (Hubscher, 2011, Journal of Hepatology, 55(3), 702-717*),* organ shortage significantly limits the application of LT. Even if sorafenib and regorafenib are applied in HCC, the overall outcome of HCC remains unsatisfactory with the median survival in early and advanced HCC of about 6-9 months and 1-2 months, respectively *(Ren et al., 2020, supra).*

Threfore, there is an important need to develop novel diagnosis tools, therapy strategy, and prognosis markers leading to patient stratification and the development of new treatment strategies for efficient therapies for HCC.

### Summary of the Invention

The present invention is directed to the finding that efficacy of clofazimine in the treatment of HCC not only correlates with the basal levels of Wnt pathway activation (the higher basal levels of the Wnt signalling, the higher would be the response to clofazimine) but is also largely influenced by the cancer mutational spectrum since either high basal levels or the Wnt signalling pathway or a high propensity to respond to Wnt3a stimulation is not sufficient for HCC cells to respond to clofazimine treatment. In particular, it has been found that responsiveness to clofazimine of HCC cell lines is also directly correlated to the presence of a genetical background due to Hepatitis B virus (HBV) infection. Therefore, the present invention allows to conduct a patient stratification for an effective treatment of responsive subjects suffering from or susceptible to develop HCC.

It is an object of the invention to provide a new therapeutic agent useful for subjects suffering from or susceptible to develop HCC.

According to a particular aspect, it is useful to provide a treatment for HCC for a specifically targeted population that would be responsive to such treatment.

Accroding to another particular aspect, due to the silencious nature of HCC development, it is advantageous to provide an early detection and treatment for HCC useful for a specifically targeted population that would be highly likely to develop a progression of HCC disease and that would responsive to such treatment.

A first aspect of the invention provides clofazimine, as well as pharmaceutically acceptable salts thereof, for use in the treatment of HCC in a subject, wherein said subject presents:
- an overactivated Wnt signalling; and
- a genetic background of Hepatitis B virus (HBV) infection.

Another aspect of the invention resides in a use of clofazimine, as well as pharmaceutically acceptable salts thereof, for the preparation of a pharmaceutical composition, in particular for the treatment of HCC in a subject as defined herein.

Another aspect of the invention relates to a pharmaceutical composition comprising clofazimine in combination with at least one agent useful in the preventing and/or treating of HCC.

Another aspect of the invention is a method for treating a subject suffering from or at risk of suffering from HCC, comprising administering clofazimine or a pharmaceutical formulation thereof in a subject in need thereof, wherein said presents:
- an overactivated Wnt signalling; and
- a genetic background of Hepatitis B virus (HBV) infection.

Another aspect of the invention is an *ex-vivo* method for detecting responsiveness to clofazimine treatment for a subject suffering from or susceptible to develop HCC.

Another aspect of the invention provides a kit for the *ex-vivo* detection of an overactivated Wnt signalling and a genetic background of Hepatitis B virus (HBV) infection in a sample, in particular from subject suffering from or at risk of suffering from HCC and the use of such a kit in a method according to the invention.

Further objects and advantageous aspects of the invention will be apparent from the claims and/or from the following detailed description of embodiments of the invention with reference to the annexed drawings.

### Brief description of the drawings

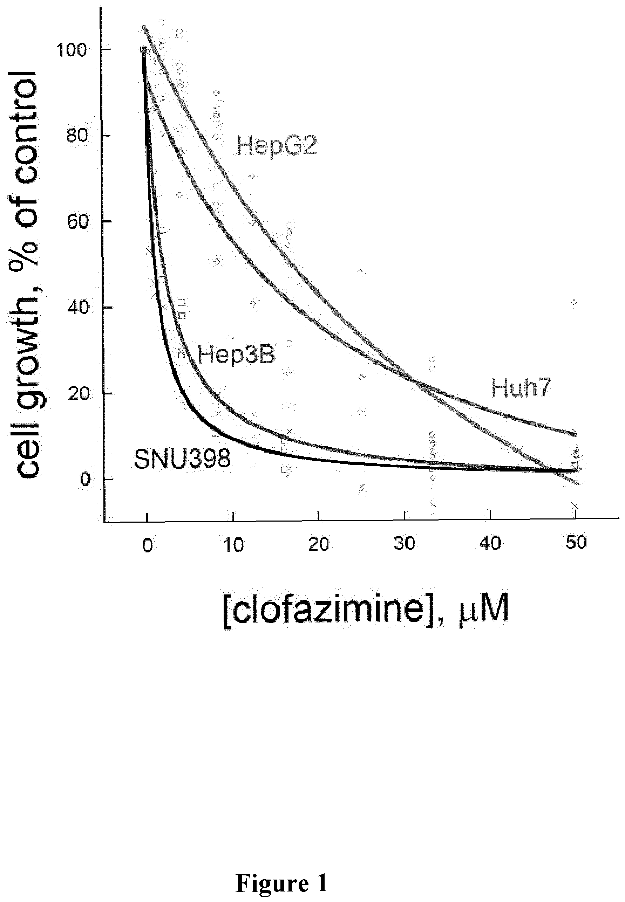

**Figure 1** shows the different sensitivities to growth inhibition by clofazimine revealed on a panel of four HCC cancer lines as described in Example 2 in a MTT assay. Of the four lines, two (SNU398 and Hep3B) have HBV genome (or parts of it) integrated in the host genome and show >10-fold stronger sensitivity to clofazimine than the two others (HepG2 and Huh7) derived from HBV-independent tumors. The curves are built on three to ten replicates for each tested clofazimine concentration.

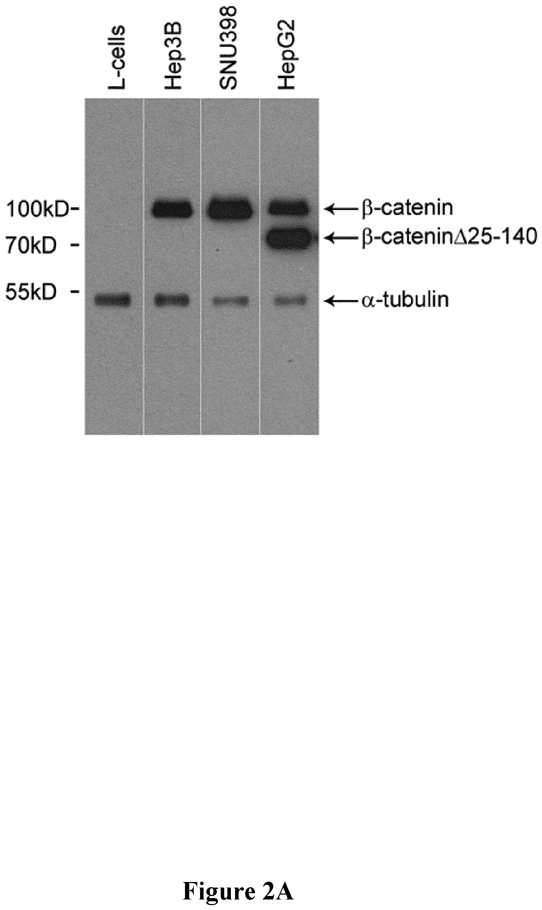

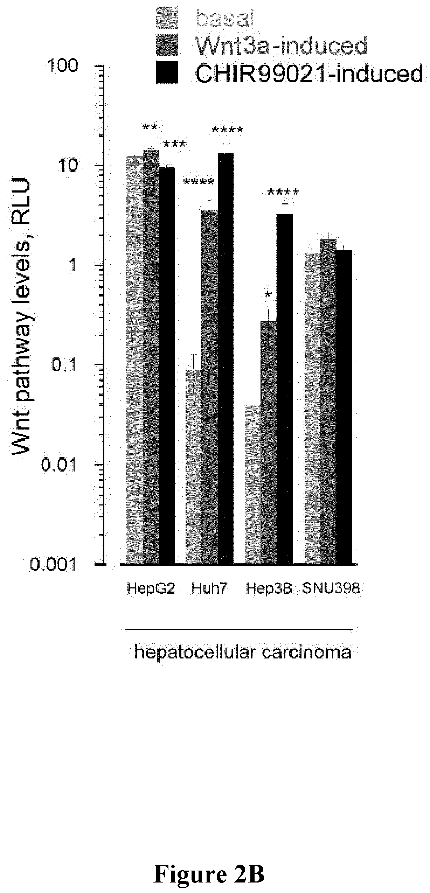

**Figure 2** represents Wnt signaling pathway levels across the four cancer lines either tested for the presence and state of total β-catenin by Western blots **(A)** or by TopFlash assay **(B)** (basal, Wnt3a-induced, and CHIR99021-induced levels) measured as described in Examples 3 and 4. Data are mean ± sem, n=3 to 20. Statistical significance is determined by the t-test, p-value < 0.05 (*), <0.01 (**), <0.005 (***), or <0.001 (****).

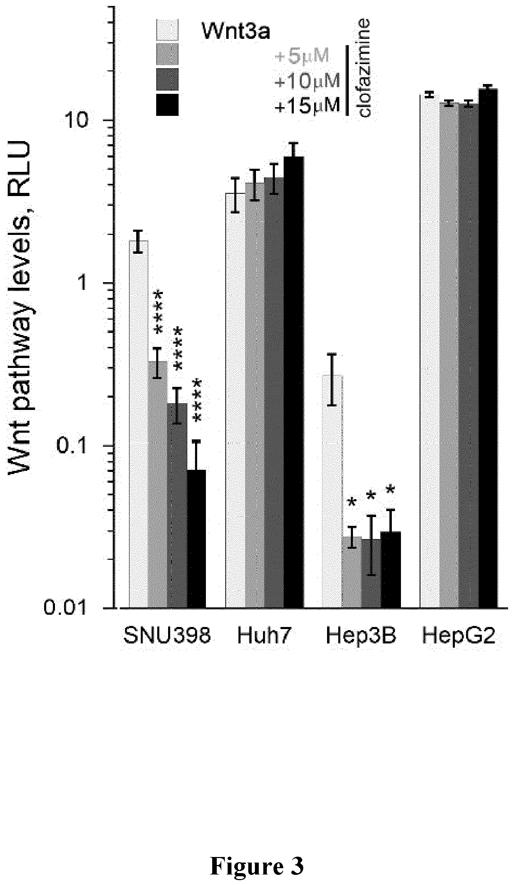

**Figure 3** represents the inhibiting effect of clofazimine on Wnt signaling across the HCC cancer lines measured as described in Example 5 when using increasing concentrations (5-10-15 µM) of clofazimine.

### Detailed Description of the invention

The term "overactivated Wnt signalling" refers to a status of cells where the basal levels of Wnt pathway activation is higher than normal and/or where the capacity of being activated by activators such as the natural agonist Wnt3a are higher. According to a particular embodiment, overactivated Wnt signalling can be detected through TopFlash assay, β-catenin stabilization assay, Dishevelled 1/2/3 phosphorylation assay, Western blot- or rtPCR-based assay monitoring levels of expression of any of Wnt pathway-target genes, β-catenin cellular immunofluorescence assay, and other assays standard in the field, to be performed on cultured cancer cells, circulating tumor cells, or tumor material from subjects suffering from HCC or susceptible to suffer from HCC, in the form of bioptate or surgically removed tumor material. For example, overactivated Wnt signalling can be detected via an increased cytonuclear β-catenin in immunohistochemistry analysis of surgical tumor samples or bioptate or circulating tumor cells or patient-derived *ex vivo* models such as patient derived cell lines. For example, measurement by Western blots of β-catenin levels in HCC cell lines can be used to detect overactivated Wnt signalling in a subject as illustrated in **Fig. 2A****.** Another example of overactivated Wnt signalling detection is TopFlash measurements **(****Fig. 2B****)** wherein *"overactivation"* means that the basal levels in the assay are above 0.01 RLU (relative luminescence units), and that the capacity of being activated by the natural agonist Wnt3a is above 0.1 RLU.

The term "a genetic background of HBV" refers to a genetic status of a cell or a subject which reveals the existence of a HBV infection or signs of past HBV infection. Such an infection or signs of past infection can be characterized by the integration of HBV DNA in the genome of a cell or presence of the HBV DNA as episome, as well as presence of HBV-specific mRNA or proteins in the cell, as detected by PCR, rt-PCR, Western blotting or immunofluorescence, or by other methods known in the art.

The term "hepatocellular carcinoma " (HHC) is a primary liver cancer which is frequently detected in pre-existing liver cirrhosis, but can also develop without such pre-conditions. It can be dected for examply by radiological characterization for example by Ultrasonography (US) as well as contrast-enhanced ultrasound (CEUS), contrast-enhanced computer tomography (CT), magnetic resonance imaging (MRI), complex biomarker analysis (including but not limited to measuring levels of alphafetoprotein in blood), or histopathological characterisation of a bioptate or surgical resection material. Subjects at risk of developping HCC can be identified by obesity and metabolic disease, chronic alcohol consumption, fatty liver disease, certain intoxications (most notably with aflatoxins), and hepatitis C virus (HCV) and hepatitis B virus (HBV) infections.

The term "subject" as used herein, refers to a human or a non-human mammal, such as non-human primate (e.g. chimpanzees and other apes and monkey species), a farm animal (e.g. cattle, sheep, pigs, goats and horses), a domestic mammal (e.g. dogs and cats), or a laboratory animal (e.g. rabbits and rodents, such as mice, rats and guinea pigs).

The term "efficacy" of a treatment according to the invention can be measured based on changes in the course of disease in response to a use or a method according to the invention. For example, the efficacy of a treatment according to the invention can be measured by its impact on signs or symptoms of illness. A response is achieved when the subject experiences partial or total alleviation, or reduction of unwanted symptoms of illness. According to a particular embodiment, the efficacy can be measured through the assessment decreased tumor growth, decreased amount of CTC, decreased number and size of metastasis, improved liver metabolic parameters, etc, which may or may not be linked with overall improvement of the patient feeling health. Further, the efficacy of a treatment according to the invention can be monitored by following the biomarkers such as levels of cytonuclear β-catenin in liver cells, HCC cells, and CTC.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease.

The term "pharmaceutical formulation" refers to preparations which are in such a form as to permit biological activity of the active ingredient(s) to be unequivocally effective and which contain no additional component which would be toxic to subjects to which the said formulation would be administered.

### Compositions

Pharmaceutical compositions of the invention contain clofazimine and a pharmaceutically acceptable carrier, diluent or excipient thereof.

According to a particular embodiment pharmaceutical compositions are oral formulations such as micronized clofazimine suspended in an oil-wax base described for Lamprene^{™} (from Novartis Pharmaceuticals Corporation) or microparticles as described in Zhang et al., 2017, Mol. Pharmaceutics, 14, 3480-3488*.*

The composition may also be provided in a powder form for reconstitution with a suitable vehicle including, but not limited to, sterile, pyrogen-free water.

Compositions of this invention may also be formulated for parenteral administration including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. Compositions of this invention may be formulated for inhalation, which may be in a form including, but not limited to, a solution, suspension, or emulsion that may be administered as a dry powder or in the form of an aerosol using a propellant, such as dichlorodifluoromethane or trichlorofluoromethane.

According to a particular embodiment, compositions according to the invention are for mucosal surface delivery.

In another particular aspect, compositions according to the invention are adapted for delivery by single or multiple administrations.

Alternatively, compositions of this invention may also be formulated as an aerosolable solution or an inhalable pharmaceutically acceptable composition. In such a formulation, the compound according to the invention is prepared for example as an inhalable dry powder or as an aerosolable solution. In particular, compositions suitable for nasal delivery may be formulated as drops, sprays, gels, suspensions, emulsions, microemulsions, micellar formulations, liposomal formulations, powders, microparticles and nanoparticles.

According to a particular embodiment, formulations according to the invention may be as described in Murashov et al., 2018, Pharmaceutics 2018,10, 238; doi:10.3390/pharmaceutics 10040238*.*

Further materials as well as formulation processing techniques and the like are set out in Part 5 of Remington's "The Science and Practice of Pharmacy", 22nd Edition, 2012, University of the Sciences in Philadelphia, Lippincott Williams & Wilkins and Remington: The Science and Practice of Pharmacy, 23rd Edition, 2020, Academic Press*,* which are incorporated herein by reference.

The invention provides compounds, compositions thereof and methods using the same useful in the treatment of HCC.

### Mode of administration

Compounds and compositions of this invention may be administered or delivered in any suitable manner.

Compounds and compositions of this invention may be administered or delivered in any manner including, but not limited to, orally, parenterally, sublingually, transdermally, transmucosally, topically, via inhalation, via buccal or intranasal administration, or combinations thereof. Parenteral administration includes, but is not limited to, intra-venous, intra-arterial, intra-peritoneal, subcutaneous and intra-muscular.

In another particular embodiment, a compound according to the invention is administered systemically orally.

The dosage administered, as single or multiple doses, to an individual will vary depending upon a variety of factors, including pharmacokinetic properties, subject conditions and characteristics (sex, age, body weight, health, and size), extent of symptoms, concurrent treatments, frequency of treatment and the effect desired.

### Combination

According to one embodiment of the invention, the compounds according to the invention and pharmaceutical formulations thereof can be administered alone or in combination with a co-agent useful in the prevention and/or treatment of a disease.

According to one aspect, compounds of the invention can be administered in combination with at least one therapeutic molecule (co-agent) useful in the prevention and/or treatment of HCC, such as agents belonging selected fromsorafenib, brinavib, sunitinib, linifanib, erlotinib, everolimus, ramicurimab, regorafenib, doxorubicin, capecitabine/ 5-fluorouracil, oxiplatin and other platinum compounds and taxanes.

According to one aspect, compounds of the invention can be administered in combination with other therapies such as radiotherapy, surgery, cryoablation, chemoembolization, liver transplantation and the like.

The invention encompasses the administration of a compound of the invention wherein the compound is administered to a subject prior to, simultaneously or sequentially with a therapeutic regimen or at least one co-agent. The compound according to the invention that is administered simultaneously with said at least one co-agent can be administered in the same or different compositions and in the same or different routes of administration

### Subjects

In an embodiment, subjects according to the invention are suffering from or at risk of suffering from HCC.

According to a further particular embodiment subjects according to the invention are suffering from HCC with a background of chronic, acute, or past HBV infection.

### Use according to the invention

The compound and methods according to the invention are useful in the treatment of HHC in a specific patient sub-group.

According to a particular aspect is provided clofazimine, as well as pharmaceutically acceptable salts thereof, for use in the treatment of HCC in a subject, wherein said subject presents:
- an overactivated Wnt signalling; and
- a genetic background of Hepatitis B virus (HBV) infection.

According to a particular embodiment, an overactivated Wnt signalling is characterized by the basal levels of Wnt pathway activation is higher than normal and/or where the capacity of being activated by activators such as the natural agonist Wnt3a are higher.

According to a particular embodiment, an overactivated Wnt signalling is characterized by increased cytonuclear β-catenin in immunohistochemistry analysis of surgical tumor samples or bioptate or circulating tumor cells or patient-derived *ex vivo* models.

According to another particular embodiment, a genetic background of Hepatitis B virus (HBV) infection is characterized by the integration of HBV DNA or it part in the genome of a cell or presence of the HBV DNA as episome, as well as presence of HBV-specific mRNA or proteins in the cell.

According to a further particular embodiment, a genetic background of Hepatitis B virus (HBV) infection is characterized by a presence of HBV genome or parts thereof in subject's cells, or presence of HBV-specific mRNA(s) in subject's cells, or presence of HBV-specific protein(s) in subject's cells.

According to another particular aspect, is provided clofazimine, as well as pharmaceutically acceptable salts thereof, for use in the treatment of HCC in a subject wherein an overactivated Wnt signalling and a genetic background of Hepatitis B virus (HBV) infection has been identified as described herein.

According to another particular aspect, is provided clofazimine, as well as pharmaceutically acceptable salts thereof, for use in a method of treatment of HHC in a subject, wherein the method comprises: **(i)** determining whether a test sample obtained from a subject is: **i1)** indicative of an overactivated Wnt signalling and **i2)** indicative of a genetic background of Hepatitis B virus (HBV) infection; and **(ii),** in the positive, administering to the patient an effective amount of clofazimine, as well as pharmaceutically acceptable salts thereof. According to another particular embodiment, a test sample for use in a method of the invention is a a tumor material that has been obtained by surgical removal, a bioptate, or circulating tumor cells (CTC) from a subject.

According to another particular embodiment, a test sample for use in a method of the invention is a sample comprising circulating tumor cells (CTC) from a subject. Examples of methods for collection and analysis of CTC from HCC patients are described in Ye, 2019, Molecular Cancer 18, 114, 1-13*; Zhang et al., 2012, 39(4), 449-460;* Wan et al., 2019, Sci Rep., 9: 18575*;* Kelley et al., 2015, BMC Cancer, 15:20*.*

According to a further particular embodiment, a test sample for identification of an overactivated Wnt signalling and a genetic background of Hepatitis B virus (HBV) infection can be a primary or stable cell culture, cancer organoid, or xenograft (e.g. mouse xenograft) obtained from a subject's tumor material.

According to another particular aspect, is provided a kit for the *ex-vivo* detection of an overactivated Wnt signalling and a genetic background of Hepatitis B virus (HBV) infection in a sample, in particular from subject suffering from or at risk of suffering from HCC. According to a further particular embodiment, said kit comprises **a)** means for the detection of overactivated Wnt signalling by TopFlash assay, β-catenin stabilization assay, Dishevelled 1/2/3 phosphorylation assay, Western blot- or rtPCR-based assay monitoring levels of expression of any of Wnt pathway-target genes, β-catenin cellular immunofluorescence assay to be performed on cultured cancer cells, circulating tumor cells, or tumor material from said subjects and **b)** means for the detection of the integration of HBV DNA or it part in the genome of a cell or presence of the HBV DNA as episome, as well as presence of HBV-specific mRNA or proteins in the cell from said sample.

According to another particular aspect, is provided a method for treating a subject suffering from or at risk of suffering from HCC, comprising administering clofazimine or a pharmaceutical formulation thereof in a subject in need thereof, wherein said presents:
- an overactivated Wnt signaling; and
- a genetic background of Hepatitis B virus (HBV) infection.

According to another particular aspect, is provided a method for treating a subject suffering from or at risk of suffering from HCC, wherein said method comprises: **(i)** determining whether a test sample from a subject is: **i1)** indicative of an overactivated Wnt signalling and **i2)** indicative of a genetic background of Hepatitis B virus (HBV) infection; and **(ii),** in the positive, administering to the patient an effective amount of clofazimine as well as pharmaceutically acceptable salts thereof.

According to one aspect, compounds of the invention can be administered at a dose of about 8 mg to about 50 mg daily, in particular from about 10 mg to about 20 mg daily (e.g. about 10 mg daily).

An *ex vivo* method of determining clofazimine treatment responsiveness from a test sample from a subject who is suffering from or at risk of suffering from HCC, said method comprising: **(i)** determining whether the test sample is: **i1)** indicative of an overactivated Wnt signalling and **i2)** indicative of a genetic background of Hepatitis B virus (HBV) infection; wherein when the test sample is not both indicative of an overactivated Wnt signalling and of a genetic background of Hepatitis B virus (HBV) infection, a responsiveness of HCC to clofazimine is low.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. The invention having been described, the following examples are presented by way of illustration, and not limitation.

### EXAMPLES

The following abbreviations refer respectively to the definitions below:
**DMEM** (Dulbecco's Modified Eagle Medium); **DMSO** (methyl sulfoxide); **FBS** (Fetal bovine serum); **MEM** (Minimum Essential Medium); **RPMI** (Roswell Park Memorial Institute).

### Example 1: Mutational spectrum of HCC cell lines is indicative of overactivated Wnt signalling

Four hepatocellular carcinoma (HCC) lines (SNU398, Hep3B, Huh7, and HepG2) have been analyzed to stratify them on their mutational profile as described below, especially looking for mutations in the genes encoding Wnt pathway components. As there is no standard list of mutations analyzed for HCC cancers, a data-driven approach and listed 10 most frequently mutated cancer-related genes (by OncoKB) identified in the HCC patient cohorts in the TCGA available from CbioPortal *(*Cerami et al., 2012, Cancer Discov 2, 401-404*)* was used. For comprehensive analysis of the relevant mutations the Broad institute Cancer Cell Line Encyclopedia (CCLE) was used *(*Next-generation characterization of the Cancer Cell Line Encyclopedia; Ghandi, et al., 2019, Nature, doi:10.1038/s41586-019-1186-3*).*

Clinical significance of the mutation was assessed with the COSMIC database (Tate et al., 2019, Nucleic Acids Res 47, D941-D947)).

The results of such analysis for the 4 cancer cell lines based on the Cancer Cell Line Encyclopedia are provided in **Table 1** below:

**Table 1**

| **Hepatocellular carcinoma lines** | | | | | |
|---|---|---|---|---|---|
| **Gene** | **Mutation frequency in patients** | | | | |
| | | **SNU398** | **Huh7** | **Hep3B** | **HepG2** |
| **TP53** | 30.80% | splice site | **Y220C** | | |
| **CTNNB1** | 26.00% | **S37C** | | | **25-140del¥** |
| **ALB** | 11.50% | | | | |
| **PCLO** | 9.40% | | C610S | | |
| **LRP1B** | 8.80% | | | | |
| **ARID1A** | 8.60% | | | | |
| **AXIN1** | 6.40% | | | **R146*** | |
| **ARID2** | 5.90% | | | | |
| **RB1** | 5.60% | | | **S576*** | |
| **KMT2D** | 5.60% | | splice site | | |

| | | | | | |
|---|---|---|---|---|---|
| *¥The in-frame deletion of amino acids 25-140 in CTNNB1 in HepG2 is not reported in the TCGA; this heterozygous deletion in HepG2 is however reported in a number of publications such as (*de La Coste et al., 1998, Proc Natl Acad Sci USA, 95, 8847-8851*) and is confirmed to exist in the line of HepG2 that was used.* | | | | | |

The mutational upregulation of the Wnt pathway is seen in three out of the four HCC lines, with pathogenic activating mutations in CTNNB1 (S37C in SNU398 and in-frame deletion of amino acids 25-140 in HepG2 *(de La Coste et al., 1998, supra)* or loss-of-function mutation in Axin1 in Hep3B cells. Thus, the mere mutational status of Wnt component genes, as applied in many other approaches to design personalized targeted anticancer treatments, would be insufficient to determine the patient group to be treated with clofazimine, and should be replaced with the criteria set above (levels of Wnt pathway compared to normal as measured in dedicated assays and analysis of the HBV status of the tumor cells.

### Example 2: Clofazimine efficiently suppresses growth of HCC cancer cell lines with a clear dichotomy between lines

The four hepatocellular carcinoma (HCC) cell lines were tested in MTT survival assay with clofazimine as described below:
50 µl of the indicated cell lines re-suspended at 60'000 cells/ml for **HepG2, Hep3B,** at 35'000/ml for **Huh7** and at 40'000/ml for **SNU398** were added into each well of a transparent 384-well plate. The cells were maintained in DMEM containing 10% FBS for Hep3B; RPMI with 10% FBS for SNU398; DMEM without glucose containing 10% FBS and 1g/L glucose for Huh7 and MEM without glucose containing 10% FBS for HepG2 and incubated at 37°C, 5% CO₂ overnight. The medium of each well was replaced by 100 µl fresh medium the next day containing the indicated concentrations of clofazimine. After incubation for 4-7 days (depending on the growth rate of line), the medium in each well was replaced by 50 µl of 0.5 mg/ml Thiazolyl blue (Carl Roth) solution in 1xPBS. The plates were incubated for 3h at 37°C. Then the solution was removed and 50 µl DMSO was added into each well. Absorbance at 570 nm was measured in a Tecan Infinite M200 PRO plate reader.

The observed effects of clofazimine demonstrated a striking dichotomy: two lines, **SNU398** and **Hep3B,** have low IC₅₀ of 1-2 µM, whereas the other two - **Huh7** and **HepG2** - are one order of magnitude less sensitive with IC₅₀s of 13 and 17µM, respectively **(****Fig. 1****).**

Therefore, those data support that it is necessary to discern the reasons behind the high sensitivity of some HCC cancer lines to clofazimine, as compared to low sensitivity of others and investigate the role of the basal levels of the Wnt pathway activity among the cell lines.

### Example 3: HCC cell lines have different basal leves of the Wnt pathway activity

In order to investigate whether the differential sensitivity between HCC cell lines to clofazimine originates from different roles the Wnt pathway plays in the oncogenic transformation which led to these cancer cell lines, a the first step was to evaluate the level of Wnt signalling in the lines by measuring the basal Wnt pathway activation in them, using the dual luciferase assay as described below and the Wnt-induced (TopFlash) and basal luciferase activity was analyzed as described (Koval et al., 2014, Biochem Pharmacol, 87, 571-578*;* Shaw et al., 2019a, Methods Cell Biol, 149, 57-75*)* (Western blots).

### Western blots

Mouse fibroblasts (L-cells) and human HCC lines Hep3B, SNU398 and HepG2 were tested for the presence and state of total β-catenin by Western blots. While L-cells do not have any basally active Wnt and resultantly no β-catenin (*Koval et al., 2014, supra),* the three HCC lines have strong levels of β-catenin indicative of their high basal levels of Wnt pathway activation **(****Fig. 2A****).** Further, the HepG2 line shows two distinct bands of the protein: the wild-type and truncated; the electrophoretic mobility of the latter corresponds to the deletion of amino acids 25-140 described for this cell line (*de La Coste et al., 1998, supra*). α-tubulin is stained on the same membrane for the loading control.

### TopFlash

Briefly, 50 µl of indicated cell lines at 84'000/ml for Huh7, or 96'000/ml for SNU398, or 144'000/ml for HepG2, Hep3B were distributed in a white opaque 384-well plate. The cells were maintained in their respective culture mediums and incubated at 37°C, 5% CO₂ overnight for attachment. Afterwards, the cells are double-transfected (1:1 mixture) with the Wnt-specific TopFlash reporter plasmid (Syngene) and a Wnt-independent CMV-Renilla plasmid monitoring the general level of cell transcription (Addgene, Cambridge, MA, USA). The relative nature of this representation (ratio of the TopFlash to the CMV-Renilla levels) permits to compare Wnt pathway levels across cell types, being independent from individual cell transfection efficiency.

Transfection was carried out as described in the manufacturer's protocol using 12 µg/ml of DNA and 40 µl/ml XtremeGENE 9 reagent (Roche). The next day, the medium of each well was replaced 30 µl fresh medium containing Wnt3a (500 ng/ml) (purified as described by (Koval and Katanaev, 2011, Biochemical Journal 433, 435) or GSK3β inhibitor (CHIR99021, Sigma-Aldrich) and clofazimine at 5, 10, and 15 µM concentrations (following 1h of preincubation with the compound). After overnight incubation, the supernatant in each well was removed and the luciferase activity was measured as described (*Koval and Katanaev, 2011, supra).*

The data show that the basal levels of Wnt pathway activation in the cancer cell lines differ from line to line dramatically, overall spanning across almost five orders of magnitude **(****Fig. 2****).** This vast range of the Wnt pathway levels agrees with the mutational spectrum of the tested cell lines **(Table 1** above). Indeed, while many cell types do not carry any mutations in the Wnt pathway components, others do. Thus, the high basal levels in the Wnt pathway activation are linked with gain-of-function mutations in CTNNB1 or with loss-of-function mutations in Axin1 **(****Fig. 2B****, Table 1)**.

### Example 4: HCC cell lines have different stimulated levels of the Wnt pathway

It was then investigated how the 4 HCC cell lines are capable to respond to activators of the Wnt pathway by two means to achieve the activation. The first is a well-studied natural agonist of the pathway, Wnt3a, known to promiscuously stimulate multiple members of the FZD receptor family (Katanaev and Buestorf, 2009, Nature Precedings, http://hdl.handle.net/10101/npre. 2009.2765.1*; Koval and Katanaev, 2011, supra).* The second is CHIR99021 - a small molecule inhibitor of GSK3β *(*Ring et al., 2003, Diabetes 52, 588-595*).* While Wnt3a-induced signaling requires the complete Wnt transduction machinery to be active, all the way from the plasma membrane to cytoplasmic and to nuclear components, CHIR99021-induced activation bypasses the plasma membrane components of the pathway (Blagodatski et al., 2014, Mol Cell Ther, 2, 28). Using these two activators, the integrity of the Wnt transduction machinery was therefore probed across the cell lines.

It can be seen that two cell lines (Huh7 and Hep3B) have relatively low basal Wnt signalling levels, strongly stimulated by addition of Wnt3a or the GSK3β inhibitor CHIR99021, while two other cell lines (HepG2 and SNU398) have very high basal levels of the Wnt pathway activation, not at all or to at best limited extent further increased by Wnt3a or CHIR99021. It is clear that the basal levels of the Wnt pathway, nor the levels achieved upon addition of either activator, do not correlate with the sensitivity of the HCC cell line to clofazimine (see Figure 1).

This analysis uncovers the broad scope of Wnt transduction capacities **(****Fig. 2****).** Certain cancer lines (SNU398, HepG2), already having high levels of the basal Wnt activation through mutations in the Wnt pathway components, do not reveal a strong stimulation of the pathway neither by Wnt3a nor by CHIR99021 (actually a decrease in the Wnt signaling levels can be induced e.g. in HepG2). Another group (HCTHuh7, Hep3B) show robust pathway stimulation by both agonists. It is clear that the basal levels of the Wnt pathway, nor the levels achieved upon addition of either activator, do not correlate with the sensitivity of the HCC cell line to clofazimine **(****Fig. 1****).**

### Example 5: Correlation between clofazimine treatment responsiveness and genetic background of Hepatitis B virus (HBV) infection

Three concentrations of clofazimine (5, 10, 15 µM) were investigated on the Wnt3a-induced response in the TopFlash assay in the panels of HCC cell lines **(****Fig. 3****).**

It can be seen that the cell lines whose Wnt signalling are inhibited by clofazimine (SNU398 and Hep3B) are the same cell lines whose growth is strongly sensitive to the drug (see **Fig. 1****).** Since the ability of clofazimine to inhibit Wnt signalling in HCC lines did not correlate with the mutational spectrum (Table 1), nor basal levels of the Wnt pathway **(****Fig. 2****),** nor stimulated levels of the Wnt pathway **(****Fig. 2****)** in these lines, it was suspected that other characteristics unique for the two clofazimine-sensitive **(****Fig. 1****)** HCC lines could be responsible for the sensitivity to the drug, both measured in the Wnt pathway **(****Fig. 3****)** and in the cell proliferation **(****Fig. 1****).** The analysis of the four HCC lines for their viral history revealed that the HBV status clearly showed that the two responsive HCC cell lines were HBV positive (Hep3B and SNU398) since integrated HBV genome was mentioned, whereas the two non-responsive were negative for the HBV genome (HepG2 and Huh7).

Therefore altogether, those data support that clofazimine treatment sensitivity of HCC cells correlates with
- an overactivated Wnt signalling; and
- a genetic background of Hepatitis B virus (HBV) infection in hepatocyte / HCC cells of a subject.

### Example 6: In vivo effect of clofazimine

The effect of clofazimine according to the invention are assayed in mouse HCC xenografts as described in Heindryckx et al., 2009, Int. J. Exp. Path., 90, 367-386 *or* Jilkova et al., 2019, Cancers, 11, 1487 . For example, one xenograft based on the clofazimine-sensitive line, and another - based on the clofazimine insensitive HCC line are compared.

## Claims

1. Clofazimine, as well as pharmaceutically acceptable salts thereof, for use in the treatment of HCC in a subject, wherein said subject presents:
- an overactivated Wnt signalling; and
- a genetic background of Hepatitis B virus (HBV) infection.

2. Clofazimine for use according to claim 1 wherein an overactivated Wnt signalling is **characterized by** increased cytonuclear β-catenin in immunohistochemistry analysis of surgical tumor samples or bioptate or circulating tumor cells or patient-derived *ex vivo* models.

3. Clofazimine for use according to claim 1 or 2 wherein a genetic background of Hepatitis B virus (HBV) infection is **characterized by** a presence of HBV genome or parts thereof in patient cells, or presence of HBV-specific mRNA(s) in subject's cells, or presence of HBV-specific protein(s) in subject's cells.

4. Clofazimine for use according to any one of claims 1 to 3, wherein an overactivated Wnt signalling and a genetic background of Hepatitis B virus (HBV) infection has been identified by TopFlash assay, β-catenin stabilization assay, Dishevelled 1/2/3 phosphorylation assay, Western blot- or rtPCR-based assay monitoring levels of expression of at least one Wnt pathway-target gene or by a β-catenin cellular immunofluorescence assay.

5. Clofazimine for use according to any one of claims 1 to 4, wherein said subject is suffering from or at risk of suffering from HCC.

6. Clofazimine for use according to any one of claims 1 to 5, wherein said subject is suffering from HCC with a background of chronic, acute, or past HBV infection.

7. Clofazimine for use according to any one of claims 1 to 6, wherein said use is in a method comprising: **(i)** determining whether a test sample from the subject is: **i1)** indicative of an overactivated wnt signalling and **i2)** indicative of a genetic background of Hepatitis B virus (HBV) infection; and **(ii),** in the positive, administering to the patient an effective amount of clofazimine, as well as pharmaceutically acceptable salts thereof.

8. Clofazimine for use according to claim 7, wherein the test sample is a tumor material that has been obtained by surgery removal, a bioptate, or circulating tumor cells (CTC) from a subject.

9. Clofazimine for use according to claim 7, wherein the test sample is a primary or stable cell culture, cancer organoid, or xenograft (e.g. mouse xenograft) obtained from a subject's tumor material.

10. Clofazimine for use according to any one of claims 1 to 9, wherein clofazimine is to be administered in combination with at least one co-agent useful in the preventing and/or treating HHC such assorafenib, brinavib, sunitinib, linifanib, erlotinib, everolimus, ramicurimab, regorafenib, doxorubicin, capecitabine/5-fluorouracil, oxiplatin and other platinum compounds and taxanes.

11. Clofazimine for use according to any one of claims 1 to 10, wherein clofazimine is to be administered in combination with radiotherapy, surgery, cryoablation, chemoembolization or liver transplantation.

12. Clofazimine for use according to any one of claims 1 to 11, wherein clofazimine is to be administered at a dose of about 8 mg to about 50 mg daily, in particular from about 10 mg to about 20 mg daily.

13. A pharmaceutical composition comprising clofazimine, in combination with at least one co-agent useful in the preventing and/or treating HHC and a pharmaceutically acceptable carrier, diluent or excipient thereof.

14. A pharmaceutical formulation according to claim 13, whereain said co-agent is an agents selected from sorafenib, brinavib, sunitinib, linifanib, erlotinib, everolimus, ramicurimab, regorafenib, doxorubicin, capecitabine/5-fluorouracil, oxiplatin and other platinum compounds and taxanes.

15. An *ex vivo* method of determining clofazimine treatment responsiveness from a test sample from a subject who is suffering from or at risk of suffering from HCC, said method comprising: **(i)** determining whether the test sample is: **i1)** indicative of an overactivated Wnt signalling and **i2)** indicative of a genetic background of Hepatitis B virus (HBV) infection; wherein when the test sample is not both indicative of an overactivated Wnt signalling and of a genetic background of Hepatitis B virus (HBV) infection, a responsiveness of HCC to clofazimine is low.
